# EUROPÄISCHE PATENTANMELDUNG

(11) **EP 2 857 093 A1**
(43) Veröffentlichungstag der Anmeldung: **08.04.2015**
(21) Anmeldenummer: 13187565.0
(22) Anmeldetag: 07.10.2013
(51) Int. Cl.: B01J 4/00, C07C 209/36, B01F 5/02

(54) **Vorrichtung und Verfahren zur Dosierung von mindestens zwei zu mischenden Flüssigkeiten**

(71) Anmelder: BASF SE, 67056 Ludwigshafen (DE)
(72) Erfinder: Die Erfindernennung liegt noch nicht vor
(74) Vertreter: Hörschler, Wolfram Johannes

(57) **Zusammenfassung**

Die Erfindung betrifft eine Vorrichtung (1) zur Dosierung von mindestens zwei zu mischenden Fluidströmen, umfassend:
- eine Mehrfachdüse (3) mit mindestens zwei einzelnen Düsen (9) zur Dosierung eines ersten Fluidstroms, wobei die einzelnen Düsen (9) der Mehrfachdüse (3) parallel zu einer zentralen Achse (7) oder geneigt in Richtung der zentralen Achse (7) ausgerichtet sind und
- eine Düse (5) zur Dosierung eines zweiten Fluidstroms, wobei die Düse (5) zur Dosierung des zweiten Fluidstroms so ausgerichtet ist, dass der zweite Fluidstrom in den die Mehrfachdüse (3) verlassenden ersten Fluidstrom dosiert wird.

Die Erfindung betrifft weiterhin ein Verfahren zur Zugabe von mindestens zwei flüssigen Fluidströmen in einen Schlaufenreaktor, wobei der Schlaufenreaktor eine Flüssigphase (15) mit darin umlaufenden Gasblasen und eine Gasphase oberhalb der Flüssigphase (15) enthält und Gas über eine Gaszufuhr im unteren Bereich des Schlaufenreaktors zugegeben wird, wobei die mindestens zwei flüssigen Fluidströme mit der Vorrichtung (1) in den Schlaufenreaktor dosiert werden, wobei die Vorrichtung (1) oberhalb der Flüssigphase (15) im Schlaufenreaktor positioniert ist, so dass sich der erste und der zweite Fluidstrom vor Auftreffen auf die Oberfläche der Flüssigphase (15) zumindest teilweise vermischen, und wobei die Vorrichtung (1) als Treibstrahldüse wirkt.

## Beschreibung

Die Erfindung betrifft eine Vorrichtung zur Dosierung von mindestens zwei zu mischenden Fluidströmen. Weiterhin betrifft die Erfindung ein Verfahren zur Zugabe von mindestens zwei zu mischenden flüssigen Fluidströmen in einen Strahlschlaufenreaktor unter Verwendung der Vorrichtung.

Strahlschlaufenreaktoren finden Einsatz insbesondere bei Verfahren, bei denen eine kontinuierliche Umsetzung von Gasen mit Flüssigkeiten durchgeführt werden soll. Geschwindigkeitsbestimmend sind hierbei zum einen der Gas-Flüssig-Stoffübergang und zum anderen auch die Wärmeabfuhr bei exothermen Reaktionen. Übliche Verfahren, bei denen ein Strahlschlaufenreaktor eingesetzt werden kann, sind zum Beispiel Hydrierungen, Oxidationen und Alkoxylierungen.

Um die Schlaufenströmung in einem Strahlschlaufenreaktor zu unterstützen, ist es üblich, am Kopf des Reaktors eine Treibstrahldüse einzusetzen. Über die Treibstrahldüse wird dabei ein Flüssigstrom in den Reaktor zudosiert. Dieser kann beispielsweise ein flüssiges Edukt oder auch zirkulierte Flüssigkeit aus dem Strahlschlaufenreaktor enthalten.

Ein entsprechender Strahlschlaufenreaktor ist zum Beispiel in WO 00/35852 beschrieben. Dieser ist als vertikal aufrechtstehender Apparat mit einer Treibstrahldüse an seinem oberen Ende ausgebildet. Im Betrieb weist der Reaktor eine interne und eine externe Zirkulationsströmung auf, wobei der Flüssigkeitsstrom der externen Zirkulationsströmung aus dem Reaktorsumpf abgezogen und über die Treibstrahldüse wieder zugeführt wird. Im Reaktor befindet sich zusätzlich ein Einsteckrohr, um die interne Zirkulationsströmung zu unterstützen. Die durch die Treibstrahldüse zugegebene Flüssigkeit führt zu einer Strömung nach unten, üblicherweise im Inneren des Einsteckrohrs. Am unteren Ende des Einsteckrohrs wird die Flüssigkeit umgelenkt und strömt außen um das Einsteckrohr wieder nach oben. Zusätzlich wird in den Ringraum außerhalb des Einsteckrohrs Gas eindosiert.

Auch aus EP-A 1 140 349 ist ein Strahlschlaufenreaktor mit einer nach unten gerichteten Treibstrahldüse im oberen Bereich bekannt.

Besonders geeignet ist ein solcher Strahlschlaufenreaktor für die Herstellung von Aminen, beispielsweise aromatischen Mono- und/oder Polyaminen durch Umsetzung der entsprechenden Nitroverbindungen mit Wasserstoff. Eine entsprechende Reaktion unter Verwendung eines Schlaufenreaktors mit einer Venturi-Düse ist beispielsweise in EP-A 634 391 beschrieben.

Nachteil der aus dem Stand der Technik bekannten Vorrichtungen zum Mischen von Fluidströmen und deren Einsatz als Treibstrahldüse in Strahlschlaufenreaktoren ist, dass die zugegebenen Flüssigkeiten schlecht miteinander vermischt werden und insbesondere bei der Herstellung von Aminen aufgrund einer zu langsamen Vermischung Ablagerungen und Überkonzentrationen entstehen können, wobei eine Überkonzentration zur Katalysatordesaktivierung führen kann.

Aufgabe der vorliegenden Erfindung ist es daher, eine Vorrichtung und ein Verfahren zur Dosierung von mindestens zwei zu mischenden Fluidströmen bereitzustellen, mit denen es ermöglicht wird, eine schnelle Vermischung der Fluidströme zu erzielen, um Ablagerungen und lokale Überkonzentrationen zu vermeiden.

Gelöst wird die Aufgabe mit einer Vorrichtung zur Dosierung von mindestens zwei zu mischenden Fluidströmen, umfassend:
- eine Mehrfachdüse mit mindestens zwei Düsen zur Dosierung eines ersten Fluidstroms, wobei die einzelnen Düsen der Mehrfachdüse parallel zu einer zentralen Achse oder geneigt in Richtung der zentralen Achse ausgerichtet sind und
- eine Düse zur Dosierung eines zweiten Fluidstroms, wobei die Düse zur Dosierung des zweiten Fluidstroms so ausgerichtet ist, dass der zweite Fluidstrom in den die Mehrfachdüse verlassenden ersten Fluidstrom dosiert wird.

Vorteil der erfindungsgemäßen Vorrichtung mit einer Mehrfachdüse und einer Düse zur Dosierung des zweiten Fluidstroms ist, dass eine gute Durchmischung erzielt werden kann und auf diese Weise lokale Überkonzentrationen vermieden werden. Zusätzlich wird durch die getrennte Zufuhr der Fluidströme und die Mischung erst hinter der Vorrichtung vermieden, dass sich innerhalb der Zuführorgane aufgrund von beginnender chemischer Reaktion Ablagerungen bilden, die zur Ausbildung von Strömungswiderständen, hohen Druckverlusten, schlechtem Mischverhalten oder völligem Verlust der Funktionalität führen können.

Durch ein schlechtes Mischverhalten können der Umsatz und/oder die Selektivität des Reaktors, in dem die Vorrichtung zur Dosierung eingesetzt wird, negativ beeinflusst werden. Zudem kann ein schlechtes Mischverhalten zu unvorteilhaften Überkonzentrationen der Edukte im Reaktor führen, was wiederum eine Katalysatordeaktivierung nach sich ziehen kann.

Um eine gute und vollständige Durchmischung zu erhalten und die Nachteile einer schlechten Durchmischung zu vermeiden, ist es besonders vorteilhaft, wenn die Vorrichtung zur Zufuhr von Fluidströmen nicht in eine Flüssigkeit eintaucht sondern - insbesondere bei Verwendung als Treibstrahldüse in einem Schlaufenreaktor - so oberhalb der Flüssigphase angeordnet ist, dass sich die zugeführten Fluidströme zumindest teilweise vermischen, bevor diese auf die Oberfläche der Flüssigphase auftreffen.

Die erfindungsgemäße Vorrichtung zur Dosierung zu mischender Fluidströme hat weiterhin den Vorteil, dass eine direkte Benetzung der einzelnen Bauteile vermieden wird, da die heißen Reaktorwände eine mögliche Zündquelle darstellen oder die Gefahr von Ablagerungen besteht. Weiterhin ist es durch die erfindungsgemäße Gestaltung auch möglich, eine teilweise oder vollständige Verblockung durch Ablagerungen in den Düsen messtechnisch zu erfassen.

Bevorzugt ist die Düse zur Dosierung des zweiten Fluidstroms eine Düse zur Dosierung des zweiten Fluidstroms, es ist jedoch auch möglich, eine Düse mit mehreren Austrittsöffnungen einzusetzen. Weiterhin besteht auch die Möglichkeit, mehrere Düsen zur Dosierung des zweiten Fluidstroms einzusetzen. Besonders bevorzugt ist der Einsatz nur einer Düse zur Dosierung des zweiten Fluidstroms.

Wenn eine Mehrfachdüse als Düse zur Dosierung des zweiten Fluidstroms eingesetzt wird oder mehrere Düsen verwendet werden, ist es besonders vorteilhaft die Fluidstrahlen so auszurichten, dass diese zu einem Strahl gebündelt werden.

In einer ersten Ausführungsform der Erfindung ist die Düse zur Dosierung des zweiten Fluidstroms außerhalb der Mehrfachdüse angeordnet und so ausgerichtet, dass der zweite Fluidstrom in Richtung der zentralen Achse der Mehrfachdüse unterhalb aller Mündungen der einzelnen Düsen der Mehrfachdüse dosiert wird.

Die Anordnung der Düse zur Dosierung des zweiten Fluidstroms außerhalb der Mehrfachdüse erlaubt eine einfache Konstruktion, da zwei voneinander unabhängige Bauteile vorliegen können. Bei der Ausrichtung der Mündungsöffnung der Düse zur Dosierung des zweiten Fluidstroms ist jedoch darauf zu achten, dass diese in Richtung der zentralen Achse der Mehrfachdüse weist, so dass ein durch die Düse zur Dosierung des zweiten Fluidstroms zudosierter Fluidstrom unterhalb der Mündungsöffnungen der Mehrfachdüse in den die Mehrfachdüse verlassenden Fluidstrom strömt und sich mit diesem vermischt. Durch die gegenüber der zentralen Achse der Mehrfachdüse geneigte Strömungsrichtung des durch die Düse zur Dosierung des zweiten Fluidstroms zudosierten Stoffstroms ergibt sich ein zusätzlicher Impuls beim Auftreffen auf den die Mehrfachdüse verlassenden Fluidstrom, so dass hierdurch eine zusätzliche verbesserte Durchmischung erzielt wird.

In einer zweiten, alternativen Ausführungsform ist die Düse zur Dosierung des zweiten Fluidstroms konzentrisch in der Mehrfachdüse angeordnet und die einzelnen Düsen der Mehrfachdüse sind in Richtung der zentralen Achse der Mehrfachdüse ausgerichtet. In diesem Fall sind ebenso wie bei der außerhalb angeordneten Düse zur Dosierung des zweiten Fluidstroms die zu vermischenden Fluidströme mit einem Winkel zueinander ausgerichtet und verlaufen nicht parallel, so dass aufgrund des zusätzlichen Impulses durch die aufeinander gerichteten Fluidströme eine verbesserte Mischung erzielt wird. Bei einer konzentrisch angeordneten Düse zur Dosierung des zweiten Fluidstroms kann die Mündungsöffnung der Düse zur Dosierung des zweiten Fluidstroms oberhalb oder unterhalb der Mündungsöffnungen der Mehrfachdüse liegen. Es ist allerdings in jedem Fall darauf zu achten, dass der Kontakt des ersten Fluidstromes mit dem zweiten Fluidstrom erst unterhalb sämtlicher Mündungsöffnungen erfolgt, um die Ausbildung unerwünschter Ablagerungen zu verhindern.

Die Mündungsöffnungen der einzelnen Düsen der Mehrfachdüse können bezogen auf die zentrale Achse der Mehrfachdüse in unterschiedlicher Höhe oder auf gleicher Höhe liegen. In einer bevorzugten Ausführungsform liegen die Mündungsöffnungen der einzelnen Düsen der Mehrfachdüse bezogen auf die zentrale Achse in unterschiedlicher Höhe. Dies hat den Vorteil, dass hydrodynamische Schwankungen des Füllstands ausgeglichen werden können.

Die Anordnung und Anzahl der einzelnen Düsen der Mehrfachdüse kann beliebig sein. Bevorzugt ist es jedoch, wenn die Mehrfachdüse mindestens drei einzelne Düsen umfasst. Weiterhin ist es vorteilhaft, wenn die einzelnen Düsen ringförmig, vorzugsweise äquidistant zueinander, um die zentrale Achse in einem oder mehreren Ringen angeordnet sind. Besonders bevorzugt weist die Mehrfachdüse drei einzelne Düsen auf, die gleichmäßig verteilt mit jeweils gleichem Abstand zur zentralen Achse um die zentrale Achse angeordnet sind.

Bei einer außerhalb der Mehrfachdüse angeordneten Düse zur Dosierung des zweiten Fluidstroms ist es weiterhin möglich, dass die Mehrfachdüse zusätzlich eine zentrale Düse umfasst.

Die Anzahl der Düsen wird dabei abhängig von der gewünschten Geschwindigkeit, dem Volumenstrom und dem Durchmesser der Mündungsöffnungen der einzelnen Düsen der Mehrfachdüse gewählt. Bevorzugt ist eine Anzahl zwischen 2 und 10 Düsen, mehr bevorzugt zwischen 2 und 8 und insbesondere zwischen 3 und 7.

Die Mündungsöffnungen der einzelnen Düsen der Mehrfachdüse können jede beliebige Form annehmen. Um keine Totzonen im Bereich der Mündungsöffnungen zu erhalten, eine mögliche Reinigung zu erleichtern und Anbackungen zu vermeiden ist es allerdings bevorzugt, wenn die Mündungsöffnungen keinen Winkel sondern nur Radien aufweisen. Besonders bevorzugt sind die Mündungsöffnungen der einzelnen Düsen der Mehrfachdüse kreisförmig oder oval.

Aus dem gleichen Grund ist auch die Mündungsöffnung der Düse zur Dosierung des zweiten Fluidstroms vorzugsweise kreisförmig oder oval.

In einer Ausführungsform der Erfindung hat mindestens eine der einzelnen Düsen der Mehrfachdüse mehrere Austrittsöffnungen. Die jeweiligen Austrittsöffnungen der einzelnen Düsen liegen dabei bevorzugt ringförmig um eine zentrale Achse der einzelnen Düse. Zusätzlich ist es auch möglich, eine konzentrische Austrittsöffnung vorzusehen. Auch hier kann die Anzahl der Austrittsöffnungen beliebig gewählt werden. Bevorzugt sind jedoch jeweils 2 bis 12 Austrittsöffnungen.

Die einzelnen Düsen der Mehrfachdüse können jeweils eine unterschiedliche Geometrie aufweisen. So ist es zum Beispiel möglich, Düsen mit nur einer Austrittsöffnung und mit mehreren Austrittsöffnungen vorzusehen und die Austrittsöffnungen zudem unterschiedlich zu gestalten. Bevorzugt ist es allerdings, die einzelnen Düsen der Mehrfachdüse so zu gestalten, dass die einzelnen Düsen der Mehrfachdüse die gleiche Öffnungsgeometrie aufweisen.

Die erfindungsgemäße Vorrichtung zur Dosierung von mindestens zwei zu mischenden Fluidströmen kann überall zum Einsatz kommen, wo mindestens zwei Fluidströme auf kurzer Strecke und schnell gemischt werden sollen. Bevorzugt sind die zu mischenden Fluidströme flüssig und die Mischung erfolgt in gasförmiger Atmosphäre. Eine schnelle Durchmischung wird unterstützt, wenn die Geschwindigkeit zumindest des die Mehrfachdüse verlassenden Fluidstroms so hoch ist, dass die Strömung turbulent ist. Eine schnelle Durchmischung von mindestens zwei Fluidströmen kann zum Beispiel für unterschiedliche chemische Reaktionen, denen die einzelnen Edukte flüssig zugegeben werden, erwünscht sein. In diesem Fall können die flüssigen Edukte mit der erfindungsgemäßen Vorrichtung in einen Reaktor eindosiert werden.

Reaktoren, in denen die erfindungsgemäße Vorrichtung zur Dosierung von mindestens zwei zu mischenden Fluidströmen eingesetzt werden kann, sind alle Reaktortypen, in denen eine Flüssigphase und eine Gasphase enthalten sind und in der Flüssigphase eine Strömung nach unten erzeugt werden soll. Geeignete Reaktoren sind zum Beispiel Schlaufenreaktoren, insbesondere Strahlschlaufenreaktoren, oder auch Kolonnen mit nach unten gerichteter Strömung mit oder ohne Einbauten, beispielsweise Blasensäulen mit Abwärtsströmung (Downflow Bubble Column).

Besonders bevorzugt wird die erfindungsgemäße Vorrichtung als Treibstrahldüse in einem Strahlschlaufenreaktor eingesetzt. In diesem Fall ist es einerseits möglich, zwei unterschiedliche flüssige Eduktströme zuzuführen oder alternativ Flüssigkeit aus dem Strahlschlaufenreaktor in einer externen Zirkulationsströmung umzupumpen und über die als Treibstrahldüse wirkende Vorrichtung zuzugeben. Die umlaufende Flüssigkeit wird in diesem Fall bevorzugt über die Mehrfachdüse zugegeben. Über die Düse zur Dosierung des zweiten Fluidstroms kann dann ein weiteres einzumischendes flüssiges Edukt zugegeben werden.

Die Erfindung betrifft weiterhin ein Verfahren zur Zugabe von mindestens zwei flüssigen Fluidströmen in einen Schlaufenreaktor, wobei der Schlaufenreaktor eine Flüssigphase mit darin umlaufenden Gasblasen und eine Gasphase oberhalb der Flüssigphase enthält und Gas über eine Gaszufuhr im unteren Bereich des Schlaufenreaktors zugegeben wird, wobei die mindestens zwei flüssigen Fluidströme mit der vorstehend beschriebenen Vorrichtung in den Schlaufenreaktor dosiert werden, wobei die Vorrichtung oberhalb der Flüssigphase im Schlaufenreaktor positioniert ist, so dass sich der erste und der zweite Fluidstrom vor Auftreffen auf die Oberfläche der Flüssigphase zumindest teilweise vermischen, und wobei die Vorrichtung als Treibstrahldüse wirkt.

Durch die Zugabe des ersten Fluidstroms über die Mehrfachdüse und die Zugabe des zweiten Fluidstroms über die Düse zur Dosierung des zweiten Fluidstroms der vorstehend beschriebenen Vorrichtung oberhalb der Flüssigphase wird eine schnelle Durchmischung der beiden Fluidströme erzielt, wobei die Durchmischung bereits zumindest teilweise, bevorzugt vollständig vor dem Auftreffen auf die Oberfläche der Flüssigphase erfolgt. Eine besonders gute Durchmischung wird erzielt, wenn die Geschwindigkeit der Fluidströme so hoch ist, dass die Strömung turbulent ist. Bei einer außerhalb der Mehrfachdüse positionierten Düse zur Dosierung des zweiten Fluidstroms ist es besonders bevorzugt, wenn der Impuls des über die Mehrfachdüse zugeführten Fluidstroms höher ist als der Impuls des über die Düse zur Dosierung des zweiten Fluidstroms zugeführten Fluidstroms, um eine gute Durchmischung zu erzielen und um weiterhin zu vermeiden, dass der über die Düse zur Dosierung des zweiten Fluidstroms zugeführte Fluidstrom den über Mehrfachdüse zugeführten Fluidstrom durchschneidet und nicht mit diesem mitgerissen wird.

Der für das erfindungsgemäße Verfahren eingesetzte Schlaufenreaktor kann in jeder beliebigen Richtung ausgerichtet sein. Bevorzugt ist jedoch eine vertikale Ausrichtung, bei der die als Treibstrahldüse wirkende Vorrichtung zur Dosierung von zu mischenden Fluidströmen im oberen Bereich angeordnet ist.

Je nach Größe des Schlaufenreaktors und Größe der Vorrichtung zur Dosierung der zu mischenden Fluidströme sowie der gewünschten Volumenströme ist es möglich, eine oder mehrere als Treibstrahldüse wirkende Vorrichtungen zur Dosierung von zu mischenden Fluidströmen einzusetzen.

In einer Ausführungsform des erfindungsgemäßen Verfahrens wird der erste Fluidstrom der Flüssigphase des Schlaufenreaktors entnommen und über die Vorrichtung wieder in den Schlaufenreaktor zurückgeführt. Hierdurch wird eine externe Zirkulationsströmung realisiert. Zudem lässt sich hierdurch der über die Düse zur Dosierung des zweiten Fluidstroms zugegebene Fluidstrom besonders gut in die Flüssigkeit einmischen und es entstehen in der Flüssigphase im Schlaufenreaktor keine Inhomogenitäten, die zu einer Reduktion des Umsatzes und/oder der Selektivität führen können oder sogar einen gegebenenfalls eingesetzten Katalysator aufgrund zu hoher Eduktkonzentration schädigen können.

Reaktionen, die in dem Schlaufenreaktor durchgeführt werden können, sind zum Beispiel Oxidationen, Hydrierungen oder Alkoxylierungen, die als Gas-Flüssig-Reaktionen durchgeführt werden.

Der erfindungsgemäße Reaktor und das Verfahren eignen sich zum Beispiel zur Herstellung von Aminen, insbesondere von aromatischen Aminen, durch Hydrierung der entsprechenden Nitroverbindungen. Hierbei enthält die Flüssigphase im Schlaufenreaktor Wasser, Katalysator, die Nitroverbindung und das Amin. Zusätzlich ist Wasserstoff in Form von Gasblasen in der Flüssigkeit enthalten, die in einer internen Zirkulationsströmung im Schlaufenreaktor umläuft. Zur Unterstützung der Zirkulationsströmung enthält der Schlaufenreaktor vorzugsweise ein Leitrohr um das die Flüssigphase strömt.

Geeignete aromatische Nitroverbindungen sind solche, die eine oder mehrere Nitrogruppen und 6 bis 18 C-Atome enthalten, beispielsweise Nitrobenzole wie Nitrobenzol, 1,3-Dinitrobenzol, Nitrotoluole wie 2,4-Dinitrotoluol, 2,6-Dinitrotoluol oder 2,4,6-Trinitrotoluol, Nitroxylole wie 1,2-Dimethyl-3-nitrobenzol, 1,2-Dimethyl-4-nitrobenzol, 1,4-Dimethyl-2-nitrobenzol, 1,3-Dimethyl-2-nitrobenzol, 2,4-Dimethyl-1-nitrobenzol und 1,3-Dimethyl-5-nitrobenzol, Nitronaphthaline wie 1-Nitronaphthalin, 2-Nitronaphthalin, 1,5-Dinitronaphthalin und 1,8-Dinitronaphthalin, Chlornitro-benzole wie 2-Chlor-1,3-dinitrobenzol, 1-Chlor-2,4-dinitrobenzol, o-Chlornitrobenzol, p-Chlornitrobenzol, m-Chlornitrobenzol, 1,2-Dichlor-4-nitrobenzol, 1,4-Dichlor-2-nitrobenzol, 2,4-Dichlor-1-nitrobenzol und 1,2-Dichlor-3-nitrobenzol, Chlornitrotoluole wie 4-Chlor-2-nitrotoluol, 4-Chlor-3-nitrotoluol, 2-Chlor-4-nitrotoluol und 2-Chlor-6-nitrotoluol, Nitroaniline wie o-Nitroanilin, p-Nitroanilin, m-Nitroanilin, Nitroalkohole wie Tris(hydroxymethyl)nitromethan, 2-Nitro-2-methyl-1,3-propandiol, 2-Nitro-2-ethyl-1,3-propandiol, 2-Nitro-l-butanol und 2-Nitro-2-methyl-1-propanol sowie beliebige Gemische aus zwei oder mehreren der genannten Nitroverbindungen.

Bevorzugt werden aromatische Nitroverbindungen, vorzugsweise Mononitrobenzol, Mononitrotoluol oder Dinitrotoluol, insbesondere 2,4-Dinitrotoluol oder dessen technische Gemische mit 2,6-Dinitrotoluol, wobei diese Gemische vorzugsweise bis zu 35 Gew.-% bezogen auf das Gesamtgemisch an 2,6-Dinitrotoluol sowie Anteilen von 1 bis 4 Gew.-% an vicinalem Dinitrotoluol und 0,5 bis 1,5 Gew.-% an 2,5 und 3,5-Dinitrotoluol aufweisen, zu den entsprechenden Aminen hydriert. Besonders wird das technische Dinitrotoluolgemisch in der Isomerenzusammensetzung eingesetzt, in der es bei der zweistufigen Nitrierung von Toluol anfällt.

Bei der Herstellung von Toluylendiisocyanat durch Hydrierung von Dinitrotoluol enthält der erste flüssige Fluidstrom, der über die Mehrfachdüse zugegeben wird, Toluylendiamin, Wasser und Katalysator und der zweite flüssige Fluidstrom, der über die Düse zur Dosierung des zweiten Fluidstroms zugegeben wird, das Dinitrotoluol.

Neben den in der mit der internen Zirkulationsströmung strömenden Flüssigkeit aufsteigenden Wasserstoffblasen enthält auch die Gasphase oberhalb der Flüssigphase Wasserstoff.

Der Schlaufenreaktor wird besonders bevorzugt in kontinuierlichen Verfahren eingesetzt. Hierbei wird ein Teil der Flüssigphase als Produkt entnommen. Bevorzugt erfolgt die Entnahme aus dem externen Zirkulationsstrom.

Besonders bevorzugt ist der Schlaufenreaktor ein Strahlschlaufenreaktor oder eine Blasensäule.

Ausführungsformen der Erfindung sind in den Figuren dargestellt und werden in der nachfolgenden Beschreibung näher erläutert.

Es zeigen:
- Figur 1A: eine Schnittdarstellung einer Vorrichtung zur Dosierung von mindestens zwei zu mischenden Fluidströmen in einer ersten Ausführungsform,
- Figur 1 B: eine Ansicht von unten auf die in Figur 1A dargestellte Vorrichtung,
- Figur 2A: eine Schnittdarstellung einer Vorrichtung zur Dosierung von mindestens zwei zu mischenden Fluidströmen in einer zweiten Ausführungsform,
- Figur 2B: eine Ansicht von unten auf die in Figur 2A dargestellte Vorrichtung,
- Figur 3A: eine Schnittdarstellung einer Vorrichtung zur Dosierung von mindestens zwei zu mischenden Fluidströmen in einer dritten Ausführungsform,
- Figur 3B: eine Ansicht von unten auf die in Figur 3A dargestellte Vorrichtung,
- Figuren 4 bis 9: verschiedene Geometrien für die Mehrfachdüse.

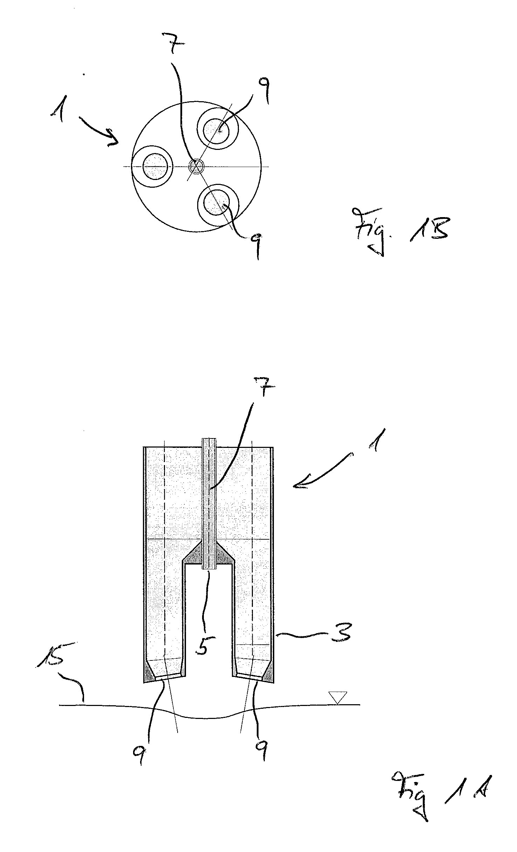

In den Figuren 1A und 1 B ist eine Vorrichtung zur Dosierung von mindestens zwei zu mischenden Fluidströmen in einer ersten Ausführungsform dargestellt.

Eine Vorrichtung 1 zur Dosierung von mindestens zwei zu mischenden Fluidströmen umfasst eine Mehrfachdüse 3 zur Dosierung eines ersten Fluidstroms und eine Düse 5 zur Dosierung des zweiten Fluidstroms zur Dosierung eines zweiten Fluidstroms.

In der in Figur 1 dargestellten Ausführungsform ist die Düse 5 zur Dosierung des zweiten Fluidstroms konzentrisch entlang einer zentralen Achse 7 der Mehrfachdüse 3 angeordnet. Hierdurch verlässt der zweite Fluidstrom im Betrieb die Düse 5 zur Dosierung des zweiten Fluidstroms in Richtung der zentralen Achse 7. Um eine gute Durchmischung mit dem ersten Fluidstrom zu erhalten, der die Mehrfachdüse 3 verlässt, weisen die einzelnen Düsen 9 der Mehrfachdüse 3 einen Winkel zur zentralen Achse 7 auf. Die einzelnen Düsen 9 der Mehrfachdüse 3 sind dabei so ausgerichtet, dass die die einzelnen Düsen 9 der Mehrfachdüse 3 verlassenden Teilströme des ersten Fluidstroms in Richtung der zentralen Achse 7 gerichtet ist. Hierdurch werden die Teilströme des ersten Fluidstroms im Betrieb in den zweiten Fluidstrom transportiert, so dass sich der erste und der zweite Fluidstrom vermischen.

Die Geschwindigkeit des ersten Fluidstroms und des zweiten Fluidstroms wird vorzugsweise so hoch gewählt, dass die Strömung turbulent ist, so dass hierdurch die Vermischung zusätzlich unterstützt wird.

In der hier dargestellten ersten Ausführungsform hat die Mehrfachdüse 3 drei einzelne Düsen 9, die gleichmäßig verteilt um die zentrale Achse 7 angeordnet sind. Hierzu ist der Abstand zwischen den einzelnen Düsen 9 jeweils gleich groß und zudem haben alle Düsen 9 den gleichen radialen Abstand zur zentralen Achse 7.

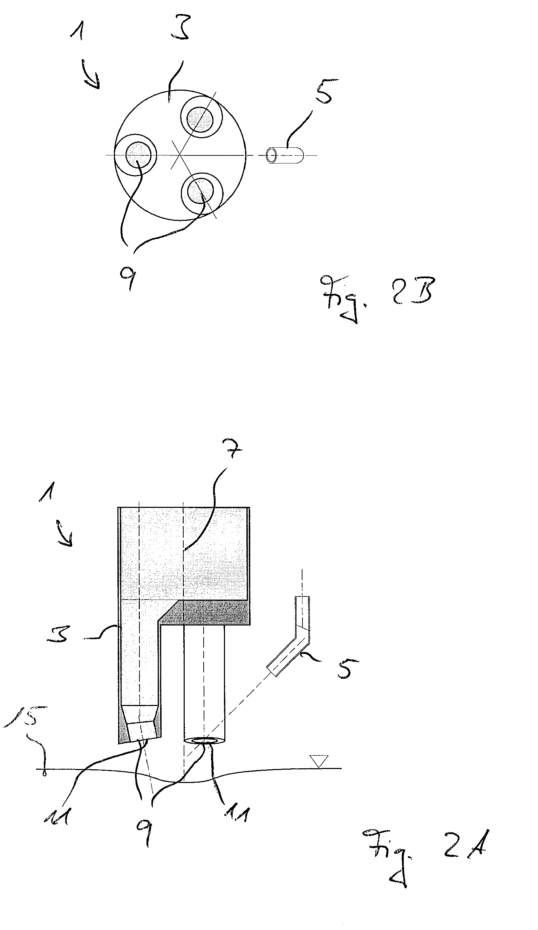

In den Figuren 2A und 2B ist eine Vorrichtung zur Dosierung zweier zu mischender Fluidströme in einer zweiten Ausführungsform dargestellt.

Im Unterschied zu der in den Figuren 1A und 1 B dargestellten Ausführungsform ist bei der in den Figuren 2A und 2B dargestellten Ausführungsform für die Vorrichtung zur Dosierung mindestens zweier zu mischender Fluidströme die Düse 5 zur Dosierung des zweiten Fluidstroms außerhalb der Mehrfachdüse 3 angeordnet. Die Düse 5 zur Dosierung des zweiten Fluidstroms ist dabei so ausgerichtet, dass der die Düse 5 zur Dosierung des zweiten Fluidstroms verlassende zweite Fluidstrom in Richtung der zentralen Achse 7 der Mehrfachdüse 3 unterhalb aller Mündungen 11 der einzelnen Düsen 9 der Mehrfachdüse 3 dosiert wird.

Die Mündungen 11 der einzelnen Düsen 9 der Mehrfachdüse 3 können, wie in Figur 2A dargestellt einen Winkel in Bezug auf die zentrale Achse 7 aufweisen. In diesem Fall werden auch die die Mündungen 11 der einzelnen Düsen 9 der Mehrfachdüse 3 verlassenden Teilströme des ersten Fluidstroms in Richtung der zentralen Achse 7 geleitet. Alternativ ist es jedoch auch möglich, dass die Mündungen der einzelnen Düsen 9 der Mehrfachdüse 3 in axialer Richtung ausgerichtet sind und somit parallel zur zentralen Achse 7 verlaufen.

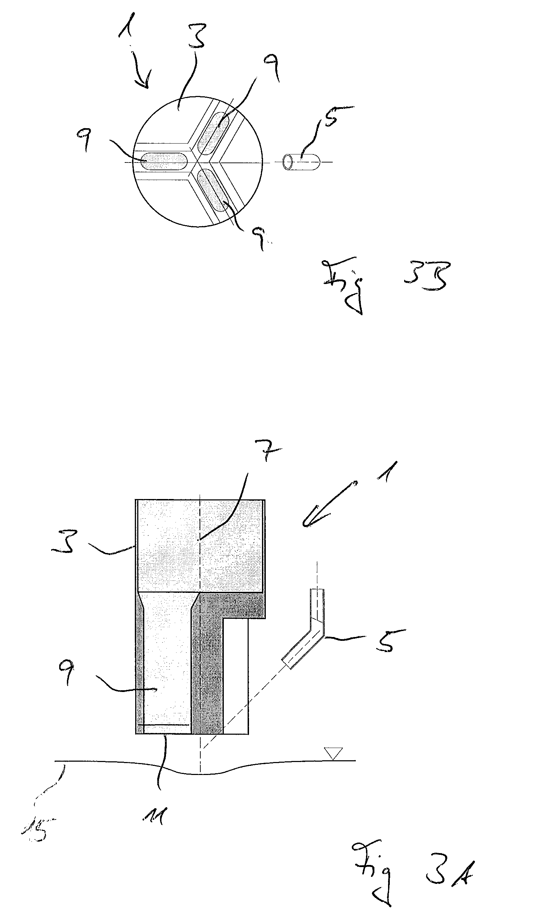

Eine entsprechende Gestaltung mit parallel zur zentralen Achse 7 verlaufenden Mündungen der einzelnen Düsen 9 der Mehrfachdüse 3 ist in den Figuren 3A und 3B dargestellt.

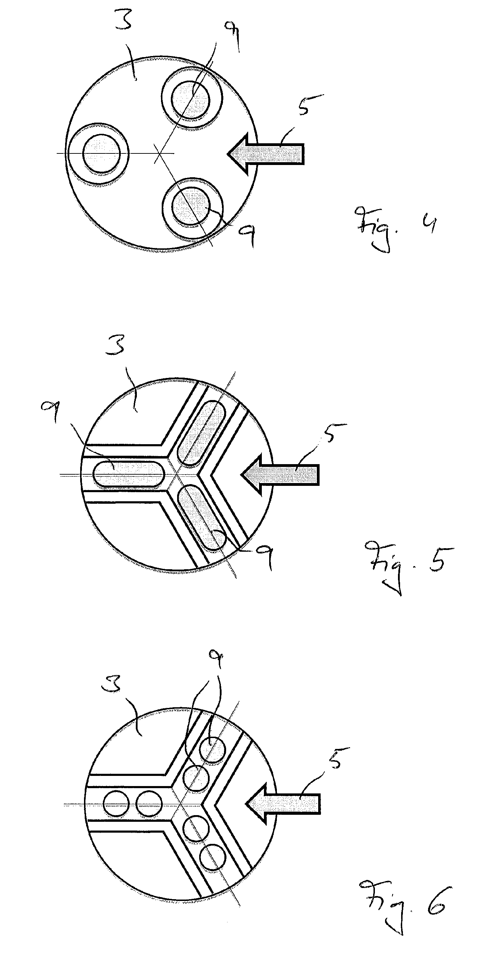

Anstelle eines kreisförmigen Querschnitts, wie in den in den Figuren 1A, 1 B, 2A und 2B dargestellten Ausführungsformen, weisen die Mündungen 11 der einzelnen Düsen 9 der Mehrfachdüse 3 einen ovalen Querschnitt auf. Neben dem hier dargestellten ovalen Querschnitt können die Mündungen jedoch auch jede beliebige andere Form annehmen. Weitere mögliche geeignete Formen für die Mündungen 11 der einzelnen Düsen 9 der Mehrfachdüse 3 sind in den Figuren 4 bis 9 dargestellt, wobei jeweils die Düse 5 zur Dosierung des zweiten Fluidstroms außerhalb der Mehrfachdüse 3 angeordnet ist.

Alternativ wäre es jeweils auch möglich, die Düse 5 zur Dosierung des zweiten Fluidstroms zentral entlang der zentralen Achse 7 zu positionieren, wie dies in den Figuren 1A und 1 B dargestellt ist. In diesem Fall ist es jeweils erforderlich, die Mündungen 11 der einzelnen Düsen 9 der Mehrfachdüse 3 geneigt auszuführen, so dass diese in Richtung der zentralen Achse 7 ausgerichtet sind, wie dies in den Figuren 1A und 2A dargestellt ist.

Bei einer Anordnung der Düse 5 zur Dosierung des zweiten Fluidstroms außerhalb der Mehrfachdüse 3, wie in den Figuren 4 bis 9 dargestellt, können die Mündungen der einzelnen Düsen 9 der Mehrfachdüse 3 entweder in axialer Richtung parallel zur zentralen Achse 7 oder alternativ - wie in den Figuren 1A und 2A dargestellt - in Richtung der zentralen Achse 7 geneigt ausgeführt sein.

In der in Figur 4 dargestellten Ausführungsform weisen die Öffnungen der einzelnen Düsen 9 der Mehrfachdüse 3 wie in den Figuren 1 B und 2B einen kreisförmigen Querschnitt auf und in der in Figur 5 dargestellten Ausführungsform einen ovalen Querschnitt, entsprechend der in Figur 3B dargestellten Ausführungsform.

Bei der in Figur 6 dargestellten Ausführungsform weisen die einzelnen Düsen 9 einen kreisförmigen Querschnitt der Mündungsöffnungen auf, wobei im Unterschied zu der in Figur 5 dargestellten Ausführungsform nicht ein ovaler Querschnitt je einzelner Düse 9 vorgesehen ist, sondern jede einzelne Düse 9 zwei in radialer Richtung nebeneinander liegende Mündungsöffnungen mit kreisförmigem Querschnitt aufweist. Alternativ zu den in Figur 6 dargestellten zwei benachbarten Mündungsöffnungen können auch noch mehr benachbarte Mündungsöffnungen vorgesehen sein. auch ist es möglich, dass die einzelnen Düsen 9 nicht nur in axialer Richtung benachbarte Mündungsöffnungen aufweisen sondern auch in radialer Richtung benachbarte. Auch ist es möglich, dass die Mündungsöffnungen einer einzelnen Düse 9 nicht in axialer Richtung benachbart sind sondern zueinander versetzt, beispielsweise zickzackförmig nebeneinander.

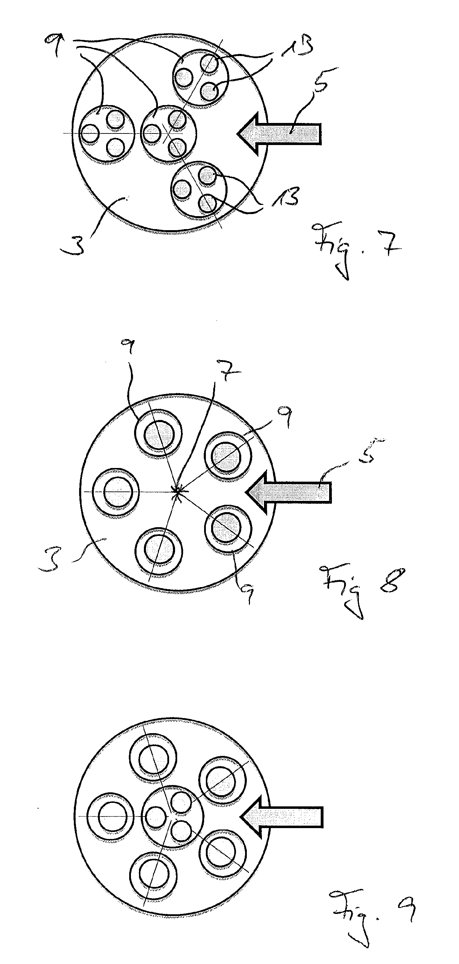

Eine Variante mit einzelnen Düsen 9 mit jeweils drei Mündungsöffnungen 13 ist beispielhaft in Figur 7 dargestellt. Hierbei ist neben den radial um die zentrale Achse 7 angeordneten einzelnen Düsen 9 eine zusätzliche einzelne Düse 9 vorgesehen, deren Mittelpunkt von der zentralen Achse 7 gebildet wird. Die Mündungsöffnungen 13 der einzelnen Düsen 9 sind in der in Figur 7 dargestellten Ausführungsform jeweils gleichmäßig verteilt um den Mittelpunkt der jeweiligen einzelnen Düse 9 angeordnet. Die Anzahl der Mündungsöffnungen 13 ist selbstverständlich nicht auf die drei hier dargestellten beschränkt. Es kann jede beliebige geeignete Anzahl an Mündungsöffnungen gewählt werden, beispielsweise bis zu 12 Mündungsöffnungen 13.

Im Unterschied zu der in Figur 4 dargestellten Ausführungsform mit drei einzelnen Düsen 9 ist in Figur 8 eine Mehrfachdüse 3 mit fünf einzelnen Düsen 9 dargestellt, wobei die einzelnen Düsen 9 auch hier jeweils äquidistant zueinander radial um die zentrale Achse 7 verteilt angeordnet sind und auch jeweils den gleichen Abstand zur zentralen Achse 7 aufweisen.

Bei der in Figur 9 dargestellten Ausführungsform ist zusätzlich zu den fünf einzelnen Düsen 9, die um die zentrale Achse 7 angeordnet sind, eine einzelne Düse 9 mit drei Mündungsöffnungen 13 vorgesehen, deren Mittelpunkt mit der zentralen Achse 7 übereinstimmt. Alternativ ist es selbstverständlich auch möglich, dass die mittlere einzelne Düse 9, deren Mittelpunkt mit der zentralen Achse 7 übereinstimmt, auch eine andere Anzahl an Mündungsöffnungen, beispielsweise eine oder zwei Mündungsöffnungen, oder auch mehr als drei, beispielsweise vier, fünf oder sechs Mündungsöffnungen aufweist.

Neben den hier dargestellten Ausführungsformen können die Mündungsöffnungen der einzelnen Düsen auch jeden beliebigen anderen Querschnitt aufweisen. Auch ist es möglich, dass die einzelnen Düsen 9 eine andere als die hier dargestellten Anzahlen an Mündungsöffnungen aufweisen. Auch kann die Anzahl der einzelnen Düsen weiter variieren. So ist es zum Beispiel auch möglich, dass die Mehrfachdüse 3 zwei, vier oder sechs einzelne Düsen 9 aufweist, die auf einer Kreislinie um die zentrale Achse jeweils äquidistant zueinander angeordnet sind. Auch ist es möglich, die einzelnen Düsen 9 mit unterschiedlichen Abständen zueinander zu positionieren oder mit unterschiedlichem Abstand der einzelnen Düsen 9 zur zentralen Achse 7. Weiterhin ist es alternativ zu den in den Figuren 1A, 2A und 3A dargestellten Varianten, bei denen die Mündungen 11 der einzelnen Düsen 9 jeweils auf gleicher axialer Höhe in Bezug auf die zentrale Achse 7 liegen, auch möglich, dass die einzelnen Düsen 9 auf unterschiedlicher Höhe enden.

Wenn mehr als zwei Fluidströme dosiert werden sollen, ist es zum Beispiel möglich, über die einzelnen Düsen 9 der Mehrfachdüse 3 unterschiedliche Substanzen zuzuführen. Je nach Anzahl der unterschiedlichen zuzuführenden Fluidströme kann die Anzahl der einzelnen Düsen 9 entsprechend variiert werden. Eine Festlegung auf drei einzelne Düsen 9 wie in den hier dargestellten Ausführungsformen ist nicht zwingend erforderlich. Auch wenn nur zwei unterschiedliche Fluidströme zugegeben werden, kann die Anzahl der einzelnen Düsen 9 der Mehrfachdüse 3, über die der erste Fluidstrom in Teilströmen zugegeben wird, von drei verschieden sein. Hier ist jede beliebige Anzahl an Düsen möglich, mit der noch eine vernünftige Zugabe und Mischung der Fluidströme erzielt werden kann und für die der Durchmesser der Mehrfachdüse 3 nicht zu groß gewählt werden muss. Sinnvoll ist eine Anzahl an einzelnen Düsen 9 in der Regel zwischen 2 und 10, bevorzugt zwischen 2 und 8 und insbesondere zwischen 3 und 7.

Wie in den Figuren 1A, 2A und 3A zu sehen, wird die Vorrichtung 1 zur Dosierung von mindestens zwei zu mischenden Fluidströmen in einem Reaktor, vorzugsweise einem Schlaufenreaktor, beispielsweise einem Strahlschlaufenreaktor, oberhalb der Flüssigphase 15 positioniert, vorzugsweise derart, dass der erste Fluidstrom und der zweite Fluidstrom in Kontakt zueinander kommen, bevor diese auf die Flüssigphase auftreffen. Besonders bevorzugt ist es, wenn der Abstand der Vorrichtung 1 zur Oberfläche der Flüssigphase 15 so groß ist, dass der erste und der zweite Fluidstrom zumindest teilweise, vorzugsweise vollständig durchmischt sind, bevor diese auf die Flüssigphase auftreffen.

In einem Schlaufenreaktor wird der Impuls der die Vorrichtung 1 verlassenden Fluidströme genutzt, um die Schlaufenströmung in der Flüssigphase zu erzeugen oder zumindest zu unterstützen.

## Patentansprüche

1. Vorrichtung zur Dosierung von mindestens zwei zu mischenden Fluidströmen, umfassend:
- eine Mehrfachdüse (3) mit mindestens zwei einzelnen Düsen (9) zur Dosierung eines ersten Fluidstroms, wobei die einzelnen Düsen (9) der Mehrfachdüse (3) parallel zu einer zentralen Achse (7) oder geneigt in Richtung der zentralen Achse (7) ausgerichtet sind und
- eine Düse (5) zur Dosierung eines zweiten Fluidstroms, wobei die Düse (5) zur Dosierung des zweiten Fluidstroms so ausgerichtet ist, dass der zweite Fluidstrom in den die Mehrfachdüse (3) verlassenden ersten Fluidstrom dosiert wird.

2. Vorrichtung gemäß Anspruch 1, **dadurch gekennzeichnet, dass** die Düse (5) zur Dosierung des zweiten Fluidstroms außerhalb der Mehrfachdüse (3) angeordnet ist und so ausgerichtet ist, dass der zweite Fluidstrom in Richtung der zentralen Achse (7) der Mehrfachdüse (3) unterhalb aller Mündungen der einzelnen Düsen (9) der Mehrfachdüse (3) dosiert wird.

3. Vorrichtung gemäß Anspruch 1, **dadurch gekennzeichnet, dass** die Düse (5) zur Dosierung des zweiten Fluidstroms konzentrisch in der Mehrfachdüse (3) angeordnet ist und die einzelnen Düsen (9) der Mehrfachdüse (3) in Richtung der zentralen Achse (7) der Mehrfachdüse (3) ausgerichtet sind.

4. Vorrichtung gemäß einem der Ansprüche 1 bis 3, **dadurch gekennzeichnet, dass** die Mündungsöffnungen (11) der einzelnen Düsen (9) der Mehrfachdüse (3) bezogen auf die zentrale Achse (7) in unterschiedlicher Höhe liegen.

5. Vorrichtung gemäß einem der Ansprüche 1 bis 4, **dadurch gekennzeichnet, dass** die Mehrfachdüse (3) mindestens drei einzelne Düsen (9) aufweist, die gleichmäßig verteilt um die zentrale Achse (7) angeordnet sind.

6. Vorrichtung gemäß Anspruch 5, **dadurch gekennzeichnet, dass** die Mehrfachdüse (3) zusätzlich eine zentrale Düse umfasst.

7. Vorrichtung gemäß einem der Ansprüche 1 bis 6, **dadurch gekennzeichnet, dass** die Mündungsöffnungen (11) der einzelnen Düsen (9) der Mehrfachdüse (3) kreisförmig oder oval sind.

8. Vorrichtung gemäß einem der Ansprüche 1 bis 7, **dadurch gekennzeichnet, dass** mindestens eine der einzelnen Düsen (9) der Mehrfachdüse (3) mehrere Mündungsöffnungen (11) hat.

9. Vorrichtung gemäß einem der Ansprüche 1 bis 8, **dadurch gekennzeichnet, dass** die Mündungsöffnungen (11) der einzelnen Düsen (9) der Mehrfachdüse (3) die gleiche Öffnungsgeometrie aufweisen.

10. Vorrichtung gemäß einem der Ansprüche 1 bis 9, **dadurch gekennzeichnet, dass** die Vorrichtung eine Treibstrahldüse in einem Strahlschlaufenreaktor ist.

11. Verfahren zur Zugabe von mindestens zwei flüssigen Fluidströmen in einen Schlaufenreaktor, wobei der Schlaufenreaktor eine Flüssigphase (15) mit darin umlaufenden Gasblasen und eine Gasphase oberhalb der Flüssigphase (15) enthält und Gas über eine Gaszufuhr im unteren Bereich des Schlaufenreaktors zugegeben wird, **dadurch gekennzeichnet, dass** die mindestens zwei flüssigen Fluidströme mit einer Vorrichtung (1) gemäß einem der Ansprüche 1 bis 10 in den Schlaufenreaktor dosiert werden, wobei die Vorrichtung (1) oberhalb der Flüssigphase (15) im Schlaufenreaktor positioniert ist, so dass sich der erste und der zweite Fluidstrom vor Auftreffen auf die Oberfläche der Flüssigphase (15) zumindest teilweise vermischen, und wobei die Vorrichtung (1) als Treibstrahldüse wirkt.

12. Verfahren gemäß Anspruch 11, **dadurch gekennzeichnet, dass** der erste Fluidstrom der Flüssigphase (15) des Schlaufenreaktors entnommen und über die Vorrichtung wieder in den Schlaufenreaktor zurückgeführt wird.

13. Verfahren gemäß Anspruch 11 oder 12, **dadurch gekennzeichnet, dass** der Schlaufenreaktor ein Strahlschlaufenreaktor oder eine Blasensäule ist.

14. Verfahren gemäß einem der Ansprüche 11 bis 13, **dadurch gekennzeichnet, dass** in dem Schlaufenreaktor eine Oxidation, eine Hydrierung oder eine Alkoxylierung durchgeführt wird.

15. Verfahren gemäß einem der Ansprüche 11 bis 13, **dadurch gekennzeichnet, dass** der Schlaufenreaktor zur Herstellung von Toluylendiamin eingesetzt wird und der erste flüssige Fluidstrom Toluylendiamin, Wasser und Katalysator enthält und der zweite flüssige Fluidstrom Dinitrotoluol.

16. Verfahren gemäß Anspruch 15, **dadurch gekennzeichnet, dass** die Gasphase oberhalb der Flüssigphase (15) Wasserstoff enthält.
